# EUROPÄISCHE PATENTSCHRIFT

(11) **EP 3 494 889 B1**
(45) Veröffentlichungstag und Bekanntmachung des Hinweises auf die Patenterteilung: **17.06.2020**
(21) Anmeldenummer: 17206434.7
(22) Anmeldetag: 11.12.2017
(51) Int. Cl.: A61B 6/03, A61B 6/00

(54) **VERFAHREN ZUR KALIBRIERUNG EINER RÖNTGENMESSEINRICHTUNG**
METHOD FOR CALIBRATING AN X-RAY MEASURING DEVICE
PROCÉDÉ D'ÉTALONNAGE D'UN DISPOSITIF DE MESURE DE RAYONS X

(43) Veröffentlichungstag der Anmeldung: 12.06.2019
(73) Patentinhaber: Siemens Healthcare GmbH, 91052 Erlangen (DE)
(72) Erfinder: Kappler, Steffen, 91090 Effeltrich (DE); Hillenbrand, Achim, 91052 Erlangen (DE)

(56) Entgegenhaltungen:
- US-A1- 2008 137 803
- US-A1- 2016 022 243
- US-A1- 2016 033 654
- US-A1- 2017 258 412

## Beschreibung

Die Erfindung betrifft ein Verfahren zur Kalibrierung einer Röntgenmesseinrichtung, welche einen spektralsensitiven Röntgendetektor umfasst, wobei ein Testobjekt im Strahlengang eines Röntgenstrahls positioniert wird, das Testobjekt durch den Röntgenstrahl bestrahlt wird, und dabei mittels der Röntgenmesseinrichtung eine Intensitätsmessung des Testobjekts durchgeführt wird, und aus der Intensitätsmessung des Testobjekts eine Absorptionsfunktion des Testobjektes ermittelt wird, wobei eine Korrekturfunktion der Absorptionsfunktion ermittelt wird, und die Röntgenmesseinrichtung anhand der Korrekturfunktion kalibriert wird.

In einem Computertomographen (CT) werden mittels Röntgenstrahlen, welche aus verschiedenen Winkelrichtungen um einen Patientenkörper herum auf diesen ausgesandt werden, verschiedene Absorptionsprofile des zu untersuchenden Körpergewebes aufgenommen, und anhand dieser Absorptionsprofile und der Kenntnis des verwendeten Strahlengangs ein Volumenmodell des Körpergewebes rekonstruiert. Vor einer Inbetriebnahme des CT, oder auch in periodischen Zeitabständen, wie z.B. bei turnusgemäßen Wartungen, wird eine Kalibrierung der Röntgenmesseinrichtung des CT durchgeführt, um allgemein Fehler in der Abbildung korrigieren zu können, welche aufgrund der Messapparatur oder aufgrund des verwendeten Messprinzips auftreten können.

Einer der bei der Kalibrierung zu berücksichtigenden Effekte ist die unter Verwendung polychromatischer Röntgenspektren auftretende sog. Strahlaufhärtung, welche zu Bildartefakten führen kann (sog. "Cupping-Artefakte"). Der Strahlaufhärtung liegt das physikalische Prinzip zugrunde, dass die Absorption energieabhängig ist, wodurch im Röntgenspektrum höherenergetische Photonen von menschlichem Gewebe oder Material mit ähnlichen optischen Eigenschaften in geringerem Maß absorbiert werden als Röntgenphotonen niedrigerer Energie. Bei der Propagation der Röntgenstrahlung durch ein Objekt wie einen Testkörper aus Wasser oder durch einen menschlichen Körper weist daher das am Röntgendetektor auftreffende Röntgenspektrum infolge der stärkeren Absorption der niederenergetischen Komponenten ein transmittiertes Spektrum mit einem höheren Mittelwert der Energie auf als das Eingangsspektrum.

Diese Strahlaufhärtung kann nun zu einer Verfälschung der Absorptionsprofile in Abhängigkeit der Dicke des im jeweiligen Absorptionsprofil untersuchten Körpergewebes führen. Insbesondere kann dabei ein Volumenelement des Materials im Inneren eines größeren Objektes einen scheinbar geringeren Absorptionskoeffizienten aufweisen, als ein vergleichbares Volumenelement an der Oberfläche des Objekts. Bei der dreidimensionalen Rekonstruktion können infolgedessen Artefakte auftreten, in welchen insbesondere ein homogenes Objekt durch den CT als inhomogen dargestellt werden kann.

Für eine Korrektur werden daher oftmals die Absorptionsprofile von Testkörpern bekannter Geometrie und insbesondere bekannter Dicke gemessen, und hieraus Korrekturfunktionen für die vom Röntgendetektor erzeugten Absorptionsdaten erstellt. Dies bedeutet jedoch einen erheblichen Aufwand bei der Berechnung der Korrekturfunktion, da für eine zufriedenstellende Korrektur der Strahlaufhärtung oftmals eine größere Anzahl an Testkörpern erforderlich ist.

Aus den US 2017/258412, US 2016/022243, US 2008/137803 und US 2016/033654 sind verschiedene Verfahren zur Kalibrierung von Röntgenmesseinrichtungen bekannt, um die Einflüsse einer Strahlaufhärtung zu korrigieren.

Der Erfindung liegt die Aufgabe zugrunde, ein Verfahren zur Kalibrierung einer Röntgenmesseinrichtung anzugeben, welches dazu geeignet ist, mit möglichst geringem Aufwand die potentiellen Einflüsse von Strahlaufhärtung auf die Messergebnisse der Röntgenmesseinrichtung zu korrigieren.

Die genannte Aufgabe wird erfindungsgemäß gelöst durch ein Verfahren zur Kalibrierung einer Röntgenmesseinrichtung, welche einen spektralsensitiven Röntgendetektor umfasst, wobei die Röntgenmesseinrichtung für eine Messung zu einer Auflösung einer Mehrzahl an unterschiedlichen Energie-Intervallen derart präpariert wird, dass durch den Röntgendetektor Energie von einfallenden Röntgenphotonen eines Röntgenstrahls in die einzelnen Energie-Intervalle aufgeteilt wird, ein Testobjekt im Strahlengang des Röntgenstrahls positioniert wird, das Testobjekt durch den Röntgenstrahl, insbesondere aus unterschiedlichen Winkeln und in unterschiedlichen Positionierungen, bestrahlt wird, und dabei mittels der Röntgenmesseinrichtung eine nach den Energie-Intervallen entsprechend aufgelöste Intensitätsmessung des Testobjekts durchgeführt wird, und anhand der Intensitätsmessung des Testobjekts eine Absorptionsfunktion des Testobjektes ermittelt wird. Hierbei ist vorgesehen, dass eines der Energie-Intervalle als ein Referenz-Intervall gewählt bzw. derart präpariert wird, dass die Absorptionsfunktion über das betreffende Enrgie-Intervall hinweg eine vernachlässigbare Energieabhängigkeit aufweist, und dass das betreffende Energie-Intervall als ein Referenz-Intervall gewählt wird, dass anhand wenigstens eines Wertes der Absorptionsfunktion im Referenz-Intervall eine Korrekturfunktion der Absorptionsfunktion für wenigstens ein weiteres Energie-Intervall ermittelt wird, und
dass die Röntgenmesseinrichtung anhand der Korrekturfunktion kalibriert wird. Vorteilhafte und teils für sich gesehen erfinderische Ausgestaltungen sind Gegenstand der Unteransprüche und der nachfolgenden Beschreibung.

Insbesondere ist die Röntgenmesseinrichtung dabei in einem CT angeordnet, oder für den bestimmungsgemäßen Betrieb in einem CT vorgesehen und entsprechend eingerichtet. Die Präparation der Röntgenmesseinrichtung für eine Messung zu einer Auflösung einer Mehrzahl an unterschiedlichen Energie-Intervallen umfasst insbesondere, dass Maßnahmen getroffen werden, welche es ermöglichen, mittels der Röntgenmesseinrichtung bei einer einmaligen Bestrahlung des Testkörpers durch den Röntgenstrahl ein resultierendes Intensitätsprofil mit der entsprechenden Energie-Auflösung registrieren zu können, und somit vorzugsweise für jedes Energie-Intervall der Röntgenstrahlung einzeln ein eigenes Intensitätsprofil zu erhalten. Insbesondere ist die nach den Energie-Intervallen entsprechend aufgelöste Intensitätsmessung als eine solche Messung implementiert, in welcher also für jedes Energie-Intervall der Röntgenstrahlung einzeln ein eigenes, bevorzugt ortsaufgelöstes Intensitätsprofil erzeugt wird.

Unter einem Testobjekt ist hierbei insbesondere ein Körper mit bekannten geometrischen Abmessungen und mit bekannten Materialeigenschaften in seinem Inneren hinsichtlich der Art und der räumlichen Verteilung umfasst, welcher bevorzugt gewisse geometrische Regularität und insbesondere eine Konvexität aufweist.

Vorzugsweise wird mittels der Röntgenmesseinrichtung eine nach den Energie-Intervallen entsprechend aufgelöste Referenz-Intensitätsmessung des Röntgenstrahls durchgeführt, wobei die Absorptionsfunktion des Testobjekts zusätzlich anhand der Referenz-Intensitätsmessung des Röntgenstrahls ermittelt wird.

Besonders bevorzugt wird hierbei die maximale Intensität I₀ gemessen, welche man erreicht, wenn der Röntgenstrahl die Röntgenmesseinrichtung ohne jegliche absorbierenden Objekte in seinem Strahlengang bestrahlt. I₀ ist somit die Referenz-Intensität bzgl. Luft. Anschließend wird die transmittierte Intensität I des Testobjektes gemessen, und hieraus eine Absorptionsfunktion a bestimmt, z.B. als das logarithmische Verhältnis der vom Testobjekt transmittierten Intensität bezogen auf die Referenz-Intensität I₀, also a = -log(I/I₀). Es sind jedoch auch andere mathematische Definitionen für die Absorptionsfunktion a denkbar, solange diese einerseits monoton in der Intensitätsmessung sind, andererseits konsistent eine gegenläufige Monotonie in der Referenz-Intensität I₀ aufweisen, und die Normbedingung a = 0 für I = I₀ erfüllen, welche der Tatsache Rechnung trägt, dass keinerlei Absorption erfolgt, wenn die gemessene Intensität gleich der Referenz-Intensität ist.

Die Präparation des betreffenden Energie-Intervalls als Referenz-Intervall kann dabei insbesondere auf der Basis von theoretischen Kenntnissen und Überlegungen hinsichtlich der in einer Messung zu erwartenden Absorptionsfunktion a' erfolgen. So kann beispielsweise aus im Energieraum hochaufgelösten Vergleichsmessungen eine Absorptionsfunktion a₊ für den Testkörper oder für einen Vergleichs-Testkörper gewonnen werden, welcher ähnliche geometrische und Materialeigenschaften aufweist, und für den Testkörper eine zu erwartende Absorptionsfunktion a' aus einer Messung, welche zu den Messungen im Verfahren vergleichbar ist, anhand einer Übertragung der gewonnenen Messresultate und der hieraus gezogenen Erkenntnisse auf den Testkörper ermittelt werden.

Aus einer Energieabhängigkeit der zu erwartenden Absorptionsfunktion a' kann nun einerseits der Wertebereich im(E) in Abhängigkeit der Energie E ermittelt werden, so dass hieraus ein reduzierter Wertebereich im_{R}(E) bestimmt werden kann, der eine vernachlässigbare Energieabhängigkeit für die entsprechende Energie der einfallenden Photonen repräsentiert, so dass beispielsweise
da'/dE ∼ 0 für E aus im_{R}(E)
gilt. Weiter kann im Energieraum auch der entsprechende Energiebereich ΔE_{R} ermittelt werden, für welchen die zu erwartende Absorptionsfunktion a' Werte in diesem reduzierten Wertebereich im_{R}(E) einnimmt. Über den so ermittelten Energiebereich ΔE_{R} ist somit die Energieabhängigkeit der zu erwartenden Absorptionsfunktion a' im Rahmen der vorliegenden Größenordnungen, welche durch den Wertebereich im(E) gegeben sind, vernachlässigbar. Das anhand der zu erwartenden Absorptionsfunktion a' bestimmte Verhalten wird nun auf die realen Messungen im Verfahren übertragen, und der ermittelte Energiebereich ΔE_{R} dabei insbesondere als das Referenz-Intervall präpariert.

Unter einem Ermitteln einer Korrekturfunktion der Absorptionsfunktion für wenigstens ein weiteres Energie-Intervall anhand wenigstens eines Wertes der Absorptionsfunktion im Referenz-Intervall ist hierbei insbesondere zu verstehen, dass zunächst wenigstens ein Wert der Absorptionsfunktion ermittelt wird, welcher einer Energie des einfallenden Röntgenphotons im Referenz-Intervall entspricht. Bevorzugt wird eine Mehrzahl an solchen Werten ermittelt. Anhand dieses oder dieser Werte wird dann für den gesamten Energiebereich eine Korrekturfunktion der Absorptionsfunktion a in Abhängigkeit derselben ermittelt, mittels derer die gemessenen Werte der Absorptionsfunktion a insbesondere außerhalb des Referenz-Intervalls im_{R}(E) zu korrigieren sind.

Aufgrund der per Konstruktion vernachlässigbaren Energieabhängigkeit der Absorptionsfunktion a über das Referenz-Intervall im_{R}(E) hinweg entspricht die Gesamtheit der Werte der Absorptionsfunktion a(ΔE_{R}) für das Referenz-Intervall ΔE_{R} einem im Wesentlichen monochromatischen Röntgenstrahl, selbst wenn real die spektrale Breite der einfallenden Röntgenstrahlung noch endlich ist, da dies in einem Frequenz- bzw. Energiebereich erfolgt, in welchem die Endlichkeit der spektralen Breite keine nennenswerten Auswirkungen auf die Absorptionsfunktion a hat. Dies bedeutet, dass über die entsprechende Präparation der Röntgenmesseinrichtung hinsichtlich des Referenz-Intervalls ein Röntgenstrahl derart gefiltert wird, dass dieser im Referenz-Intervall ΔE_{R} für das Verhalten der Absorptionsfunktion a dieselbe Wirkung aufweist, wie ein monochromatischer Röntgenstrahl.

Infolge der Energieabhängigkeit der Absorption von Röntgenphotonen in einem Material ist jedoch die Intensität eines durch einen Testkörper transmittierten monochromatischen Röntgenstrahls bzw. dessen Absorption nur noch durch eine exponentielle Abschwächung charakterisiert, welche lediglich von der Dicke des für den Röntgenstrahl zu durchdringenden Materials abhängt. Diese exponentielle Abschwächung kann dabei für unterschiedliche Materialien in ihrer Eindringtiefe variieren, es tritt also im Exponenten ein materialabhängiger Vorfaktor zur Dicke als eigentlicher Variable hinzu, jedoch ist die funktionale Abhängigkeit hier eindeutig vorgegeben. Bevorzugt wird durch die Definition der Absorptionsfunktion a dieser Zusammenhang für monochromatische Röntgenphotonen adäquat berücksichtigt, wie beispielsweise in der oben angegebenen über den Logarithmus der Intensitäten, so dass die Absorptionsfunktion a in diesem Fall linear von der Dicke abhängt.

Das Ermitteln der Korrekturfunktion kann dabei konkret derart erfolgen, dass für jedes Ortsauflösungs-Pixel der Röntgenmesseinrichtung die gemessenen Intensitäten zu verschiedenen Messungen des Testkörpers, bevorzugt mit jeweils verschiedenen Dicken des durchstrahlten Materials des Testkörpers, in einen sog. Scatter-Plot eingetragen werden, in welchem z.B. der Abszissenwert die gemessene Absorptionsfunktion a ist, während der Ordinatenwert gegeben ist durch die bei der entsprechenden Dicke für einen monochromatischen Röntgenstrahl angenommene Absorptionsfunktion A (nicht zu verwechseln mit der zu erwartenden Absorptionsfunktion a' bei der Ermittlung des Monotonieverhaltens der Absorptionsfunktion a). Aus den derart generierten Punkten bzw. Wertepaaren im Scatter-Plot kann nun eine Korrekturfunktion der Absorptionsfunktion a in Abhängigkeit derselben derart bestimmt werden, dass die korrigierte Absorptionsfunktion A(a) derjenigen entspricht, welche man für monochromatische Röntgenphotonen erhalten würde. Das Bestimmen der Korrekturfunktion aus den genannten Punkten bzw. Wertepaaren im Scatter-Plot kann dabei über in der statistischen Datenverarbeitung bekannte Verfahren erfolgen, z.B. mittels einer polynomialen Glättung.

Anhand der Korrekturfunktion bzw. der korrigierten Absorptionsfunktion A(a) kann nun die Röntgenmesseinrichtung derart kalibriert werden, dass Effekte der Strahlaufhärtung dabei korrigiert werden, und insbesondere die Hounsfield-Skala zur Normierung uneingeschränkt eingehalten werden kann.

In einer vorteilhaften Ausgestaltung der Erfindung wird als Referenz-Intervall ein breitbandiges Energie-Intervall präpariert, dessen Untergrenze wenigstens den Wert von 60%, bevorzugt wenigstens 2/3 der Maximalenergie des Spektrums des Röntgenstrahls beträgt, und dessen spektrale Breite bevorzugt wenigstens 1/5, besonders bevorzugt wenigstens 1/4 der Maximalenergie des Spektrums des Röntgenstrahls beträgt. Die Maximalenergie des Spektrums des Röntgenstrahls ist hierbei insbesondere beschränkt durch die Beschleunigungsspannung der Anode der die Röntgenstrahlung erzeugenden Röntgenröhre.

Ein derartiges Vorgehen trägt einerseits der Tatsache Rechnung, dass übliche Röntgenmesseinrichtungen, wie z.B. quantenzählende Röntgendetektoren, meist im Energieraum nur eine endliche Auflösung im Umfang von beispielsweise vier bis sechs Energie-Intervallen aufweisen, und daher im für den bestimmungsgemäßen Betrieb eingerichteten Zustand angesichts des Röntgenspektrums von ca. 10 keV bis weit über 100 keV jeweils eine merkliche spektrale Breite aufweisen. Andererseits wird hierbei der Umstand ausgenutzt, dass für die höherenergetischen Röntgenphotonen im angegebenen spektralen Bereich bereits keine weitere nennenswerte Strahlaufhärtung mehr bei der Transmission bzw. Absorption im Testkörper und auch im Patientengewebe stattfindet, und somit die Absorptionsfunktion in diesem Bereich keine nennenswerte Energieabhängigkeit mehr zeigt, wodurch das gesamte breitbandige Energie-Intervall als Referenz-Intervall verwendet werden kann.

Günstigerweise wird dabei als Referenz-Intervall ein für den energieaufgelösten Normalbetrieb der Röntgenmesseinrichtung vorgesehenes Energie-Intervall gewählt. Dies hat den Vorteil, dass die Präparation der einzelnen Energie-Intervalle für die Messungen zum Bestimmen der Korrekturfunktion gleich im Rahmen einer Präparation für die Inbetriebnahme durchgeführt werden kann.

In einer weiter vorteilhaften Ausgestaltung der Erfindung wird als Referenz-Intervall ein schmalbandiges Energie-Intervall präpariert, dessen spektrale Breite höchstens 1/8, bevorzugt höchstens 1/10 der Maximalenergie des Spektrums des Röntgenstrahls beträgt. Dies bedeutet insbesondere, dass eigens für die Messungen zum Bestimmen der Korrekturfunktion ein entsprechendes Energie-Intervall als Referenz-Intervall präpariert wird, mittels dessen die Korrekturfunktion für die anderen Energie-Intervalle, wie sie für den energieaufgelösten Normalbetrieb der Röntgenmesseinrichtung vorgesehen sind, zur entsprechenden Kalibrierung ermittelt werden. Bevorzugt werden dabei Messungen zu mehreren Referenz-Intervallen von vergleichbarer spektraler Breite und unterschiedlicher Positionierung im Energiebereich durchgeführt, um für das gesamte Röntgenspektrum eine besonders gute Energie-Auflösung und eine hohe Präzision im Energieraum zu erhalten.

Zweckmäßigerweise wird dabei für den energieaufgelösten Normalbetrieb der Röntgenmesseinrichtung die Präparierung des Referenz-Intervalls aufgehoben. D.h., nach den Messungen zum Ermitteln der Korrekturfunktion und einer entsprechenden Kalibrierung der einzelnen Energie-Intervalle der Röntgenmesseinrichtung für den energieaufgelösten Normalbetrieb findet sich das Referenz-Intervall nicht mehr in der Konfiguration der Energie-Intervalle.

Als vorteilhaft erweist es sich weiter, wenn ortsaufgelöst Werte der Absorptionsfunktion für das Referenz-Intervall gegen Werte der Absorptionsfunktion für das wenigstens eine weitere Energie-Intervall aufgetragen werden, und hieraus die Absorptionsfunktion für das Referenz-Intervall in Abhängigkeit der Absorptionsfunktion A(a) für das wenigstens eine weitere Energie-Intervall als Korrekturfunktion im betreffenden Energie-Intervall ermittelt wird. Insbesondere bedeutet dies, dass für jedes Ortsauflösungs-Pixel der Röntgenmesseinrichtung eine entsprechende Auftragung erfolgt. Dies kann insbesondere in der Form eines Scatter-Plots erfolgen, wobei die Korrekturfunktion anhand statistischer Methoden, wie z.B. polynomialer Glättung, aus den so erzeugten Daten ermittelt werden kann. Auf diese Weise lässt sich besonders einfach ein funktionaler Zusammenhang zwischen den real durch die Röntgenmesseinrichtung ermittelten Messungen in den verschiedenen Energie-Intervallen und einer hypothetischen Messung für monochromatische Röntgenstrahlung herstellen, und dieser funktionale Zusammenhang dann für die Korrektur verwenden.

In einer weiter vorteilhaften Ausgestaltung führt die Röntgenmesseinrichtung für die Intensitätsmessung des Testobjekts eine Rotationsbewegung bezüglich des Testobjekts durch. Insbesondere führt auch die Strahlungsquelle des Röntgenstrahls eine Rotation durch, und bevorzugt sind beide Rotationen derart zueinander synchronisiert, dass die Röntgenmesseinrichtung und Röntgenstrahlquelle keine Relativbewegung zueinander durchführen. Insbesondere wird hierbei durch die Rotation die Weglänge, welche der Röntgenstrahl durch das Testobjekt propagiert, variiert. Hierdurch lassen sich mittels nur eines Testobjektes anhand der Kenntnis dieser Weglänge Werte der Absorptionsfunktion zu verschiedenen Dicken des Testobjektes gewinnen, ohne hierfür eine aufwendige Positionierung von mehreren Testobjekten durchführen zu müssen. In einem CT ist dies aufgrund der Notwendigkeit der Rekonstruktion des Volumenmodells aus den Absorptions- bzw. Intensitätsdaten und einer entsprechend erforderlichen Kalibrierung über verschiedene Dicken des Testobjektes besonders vorteilhaft.

Günstigerweise wird als Röntgenmesseinrichtung ein quantenzählender Röntgendetektor kalibriert. Ein derartiger Röntgendetektor erlaubt eine energieaufgelöste Detektion von Röntgenphotonen bei hoher Ortsauflösung, wobei jedoch für die korrekte Normierung der Zählereignisse hinsichtlich der Referenz-Intensität I₀ ohne Objekt im Strahlengang eine aufwändige Kalibrierung notwendig ist, für welche das vorgeschlagene Verfahren besonders gut geeignet ist.

Als weiter vorteilhaft erweist es sich, wenn das Referenz-Intervall über wenigstens eine Spannungsstufe in einem Signalverstärker der Röntgenmesseinrichtung präpariert wird. Die ortsaufgelöste Detektion von Röntgenphotonen kann auf unterschiedliche Art erfolgen. Eine Möglichkeit ist es dabei, generell die Energie der einfallenden Röntgenphotonen durch Ionisierung von Atomen in einem hierfür vorgesehenen Kristall, beispielsweise einem Halbleiter, in elektrische Ladungen und weiter in Strom- oder Spannungssignale umzuwandeln. Bevorzugt ist dabei die Amplitude des entsprechenden Strom- oder Spannungssignals proportional zur Energie eines jeweils einfallenden Röntgenphotons. Die Filterung im Detektor nach einzelnen Energie-Bereichen kann dann anhand der genannten Spannungsstufen erfolgen, z.B. im Sinne von "Bias-Spannungen", gegen welche das erzeugte Strom- oder Spannungssignal verlaufen muss, so dass diejenigen Signale von Röntgenphotonen einer niedrigeren Energie als die der gewählten Bias-Spannung entsprechenden die Bias-Spannung nicht überwinden, und somit das Signal entsprechend nicht registriert wird. Über eine Mehrzahl an Spannungsstufen lässt sich somit der Energiebereich in eine Mehrzahl an Intervallen aufteilen.

Die Erfindung nennt weiter ein bildgebendes medizinisches Gerät mit wenigstens einer Röntgenquelle zur Erzeugung eines Röntgenstrahls sowie einer Röntgenmesseinrichtung, welches zur Kalibrierung der Röntgenmesseinrichtung gemäß dem vorbeschriebenen Verfahren eingerichtet ist. Die für das Verfahren zur Kalibrierung und für seine Weiterbildungen angegebenen Vorteile können dabei sinngemäß auf das bildgebende medizinische Gerät übertragen werden.

Insbesondere für ein bildgebendes medizinisches Gerät, in welchem ein spektralsensitiver Röntgendetektor zur 3D-Rekonstruktion verwendet wird, ist das beschriebene Verfahren hierbei von Vorteil. Das bildgebende medizinische Gerät kann demnach insbesondere als ein CT, aber auch als ein C-Bogen-Gerät ausgestaltet sein.

Nachfolgend wird ein Ausführungsbeispiel der Erfindung anhand einer Zeichnung näher erläutert. Hierbei zeigen jeweils schematisch:
- FIG 1: in einer Querschnittdarstellung ein CT, in welchem ein Testkörper zur Kalibrierung eines Röntgendetektors positioniert ist,
- FIG 2: in einem Diagramm für ein Pixel des Röntgendetektors nach FIG 1 eine Referenz-Intensitätsmessung und eine Messung der vom Testkörper transmittierten Intensität,
- FIG 3: in einem Blockdiagramm ein Verfahren zur Kalibrierung des Röntgendetektors nach FIG 1.

Einander entsprechende Teile und Größen sind in allen Figuren jeweils mit gleichen Bezugszeichen versehen.

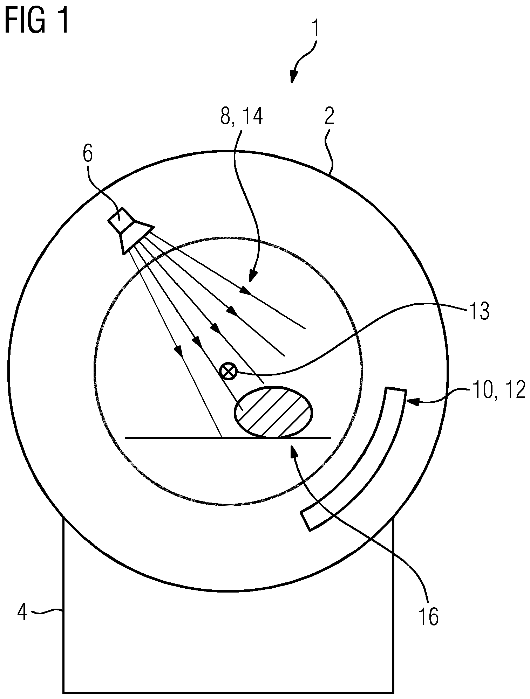

In FIG 1 ist schematisch in einer Querschnittdarstellung ein CT 1 gezeigt, welches einen Drehkranz 2 und einen Halterahmen 4 aufweist. Auf dem Drehkranz 2 sind eine Röntgenquelle 6 zur Erzeugung eines Röntgenstrahls 8 und eine Röntgenmesseinrichtung 10, welche als quantenzählender Röntgendetektor 12 ausgebildete ist, angeordnet. Im Betrieb des CT 1 rotiert nun der Drehkranz 2 um eine Achse 13 senkrecht zur Bildebene, wobei ein im Inneren des Drehkranzes 2 positionierter Patient aus verschiedenen Winkelrichtungen vom Röntgenstrahl 8 durchdrungen wird, so dass zu jeder dieser Winkelrichtungen durch den Röntgendetektor 12 ein zweidimensionales Absorptionsprofil der Röntgenstrahlung erzeugt wird, welches an eine nicht näher dargestellte Bildverarbeitungseinheit auf dem Halterahmen 4 übertragen wird. Aus der Gesamtheit der zu allen Winkelrichtungen erzeugten Absorptionsprofile wird dann über eine Rücktransformation ein dreidimensionales Volumenmodell des im CT untersuchten Patientengewebes erzeugt.

Infolge der Energieabhängigkeit der Absorption von Röntgenstrahlung durch alle Arten von Körpergewebe und auch Wasser, und der hierdurch entstehenden Strahlaufhärtung, also der Verschiebung des Röntgenspektrums durch ein im Strahlengang 14 des Röntgenstrahls 8 positioniertes Objekt hin zu im Mittel höherenergetischen Röntgenphotonen, sind hierbei die einzelnen Absorptionsprofile hinsichtlich der Strahlaufhärtung zu korrigieren, damit die tatsächlich ermittelte Absorption für eine Winkelrichtung keine durch die Dicke des absorbierenden Gewebes bedingten Verzerrungen enthält. Üblicherweise werden für eine derartige Korrektur Kalibrationsmessungen der Absorptionsprofile von einer Mehrzahl an Testobjekten von bekannter Geometrie und Materialbeschaffenheit durchgeführt, um anhand der Kenntnis der Dicke des durchstrahlten Testobjektes für eine Winkelrichtung zusätzliche Informationen hinsichtlich einer erwarteten Absorptionsverteilung zu erhalten.

Vorliegend wird nun ein anderer Weg eingeschlagen, welcher nur die Messung eines einzigen Testobjektes 16 erfordert, wie anhand der folgenden Figuren beschrieben ist.

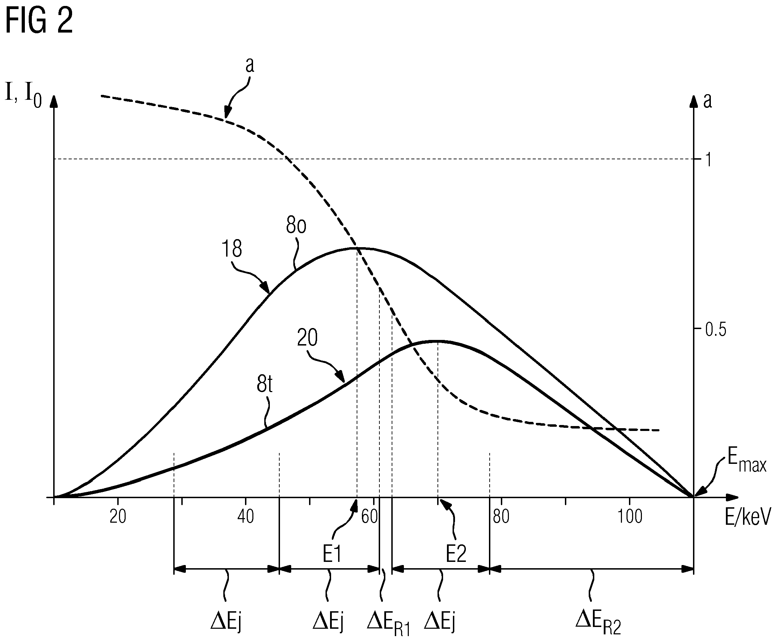

In FIG 2 sind schematisch in einem Diagramm gegen die Energie E der einfallenden Röntgenphotonen die Intensität I₀ eines Röntgenstrahls 8o ohne Filterung durch ein Testobjekt sowie die Intensität I eines durch ein Testobjekt 16 nach FIG 1 teilweise absorbierten Röntgenstrahls 8t für ein Pixel des Röntgendetektors 12 dargestellt. Die Werte für die einzelnen Energien hängen maßgeblich vom verwendeten Anodenmaterial der Röntgenquelle ab. Zur besseren Übersichtlichkeit wurde hierbei auf die Darstellung der Spektralbeiträge des für das Anodenmaterial charakteristischen Linienspektrums verzichtet.

Die Intensität I₀ des ungefilterten Röntgenstrahls 8o weist zunächst einen monotonen Anstieg 18 in der Energie E auf, um bei einer Energie E₁ von knapp über 55 keV ihr Maximum einzunehmen und dann monoton abzufallen. Die Intensität I des vom Testobjekt 16 gefilterten Röntgenstrahls 8t weist infolge der höheren Absorption bei niedrigeren Energien einen zunächst weitaus flacheren Anstieg 20 auf, um dann das Maximum bei einem Wert E₂ von ca. 70 keV einzunehmen. Auch wird aus dem Diagramm von FIG 2 deutlich, dass die wesentlichen Beiträge der Intensität I für den Röntgenstrahl 8t generell von der Intensität I₀ des Röntgenstrahls 8o ausgehend zu höheren Energien hin verschoben sind.

Weiter ist zur besseren Illustration in FIG 2 die energieaufgelöste Detektion angedeutet, wie sie vom Röntgendetektor 12 nach FIG 1 erfasst und weitergegeben wird. Der Röntgendetektor teilt dabei die Energie E in einzelne Energie-Intervalle ΔEⱼ auf. Sämtliche Röntgenphotonen, deren Energie in einem derartigen Energie-Intervall ΔEⱼ liegt, weisen folglich für den Röntgendetektor 12 im Rahmen von dessen energetischem Auflösungsvermögen, welches genau durch die Energie-Intervalle ΔEⱼ gegeben ist, die gleiche Energie auf. Die Intensitäten I, I₀ werden dann nur noch als vorliegend nicht dargestellte Blöcke I(ΔEⱼ), I₀(ΔEⱼ) erfasst.

Eine Absorptionsfunktion a := -log(I/I₀), eingezeichnet als gestrichelte Linie mit Skalierung an der rechten Achse - weist nun offenkundig infolge der Energieabhängigkeit der beteiligten Intensitäten I, I₀ seinerseits eine entsprechende Energieabhängigkeit auf. Durch die grobe Einteilung der Energie in einzelne Intervalle ΔEⱼ würde die korrekte Erfassung der Energieabhängigkeit in der Absorptionsfunktion a verlorengehen, sofern diese Energieabhängigkeit der Absorptionsfunktion a, wie vorliegend für die Kalibrierung des Röntgendetektors 12 der Fall, von Belang ist und nicht vernachlässigt werden kann.

Ein Energie-Intervall ΔE_{R1} wird jedoch derart schmal gewählt - vorliegend von 60 bis 62 keV - dass die Absorptionsfunktion a sich über das besagte Energie-Intervall ΔE_{R1} hinweg nur um ca. 0,05 bei einem Wertebereich von ca. 0,2 bis ca. 1,2, also kaum nennenswert ändert. Für dieses Energie-Intervall ΔE_{R1} ist also der Röntgenstrahl 8 hinsichtlich des Verhaltens der Absorptionsfunktion a im Wesentlichen monochromatisch, so dass es als Referenz-Intervall bei Messungen herangezogen werden kann, in denen die Kenntnis der Absorptionsfunktion für einen monochromatischen Röntgenstrahl erwünscht ist.

Je nach Anwendung kann das Energie-Intervall ΔE_{R1} jedoch auch breiter gewählt werden, wobei die Wahl vom Verhalten der Absorptionsfunktion a abhängt. So kann beispielsweise angesichts der Maximalenergie Eₘₐₓ des Spektrums des Röntgenstrahls 8 von ca. 110 keV das Referenz-Intervall ΔE_{R1} über den Bereich von ca. 70-80 keV auch mit einer spektralen Breite von ca. 10-11 keV gewählt werden, da dort die Absorptionsfunktion a ihre Werte im Vergleich zum gesamten Wertebereich nur unwesentlich ändert.

Eine weitere Möglichkeit für ein Energie-Intervall ohne nennenswerte Energieabhängigkeit der Absorptionsfunktion a, und somit eine weitere Möglichkeit eines Referenz-Intervalls, ist durch das Energie-Intervall ΔE_{R2} am hochenergetischen Rand des Spektrums gegeben. Hierbei weist das Energie-Intervall ΔE_{R2} eine nicht unerhebliche spektrale Breite von mehreren zehn keV auf, jedoch fällt diese spektrale Breite infolge des Verhaltens der beiden Intensitäten I, I₀ beim Verlauf der Absorptionsfunktion a nicht nennenswert ins Gewicht, weshalb auch das Energie-Intervall ΔE_{R2} als Referenz-Intervall verwendet werden kann.

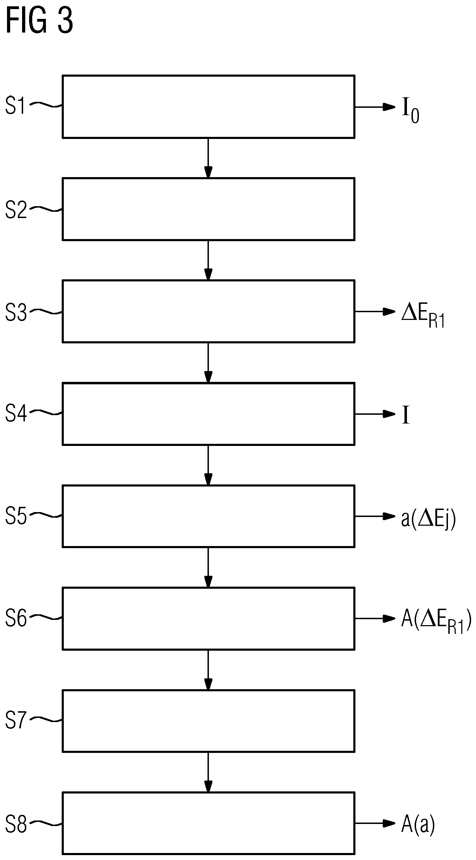

Dieses Wissen kann nun dafür verwendet werden, eine Korrekturfunktion zur im Betrieb real gemessenen Absorptionsfunktion zu ermitteln. In FIG 3 hierzu ist schematisch anhand eines Blockdiagramms der Ablauf eines Verfahrens zur Kalibrierung des Röntgendetektors 12 des CT 1 nach FIG 1 dargestellt. In einem ersten Schritt S1 wird eine Referenz-Messung der Intensität I₀ des nur durch die Luft im Innenraum des CT gefilterten Röntgenstrahls 8o durchgeführt. Als nächstes wird in einem Schritt S2 das Testobjekt 16 im Strahlengang 14 des Röntgenstrahls 8 positioniert. Hierfür ist das Testobjekt bevorzugt exzentrisch zu lagern, so dass zu verschiedenen Winkelrichtungen erzeugte Röntgenstrahlen 8t eine verschiedene Weglänge durch das Testobjekt 16 zurücklegen müssen, was bei der Berechnung der Korrekturfunktion berücksichtigt werden kann.

Im nachfolgenden Schritt S3 wird nun über ein Einstellen entsprechender Spannungsstufen in der Vorverstärkung für die erzeugten Signals ein Energie-Intervall ΔEⱼ als Referenz-Intervall ΔE_{R1} vorbereitet. Im vorliegenden Fall wird hierbei ein schmalbandiges Energie-Intervall gewählt. Im anschließenden Schritt S4 wird eine Messung der Intensität I des von Testobjekt 16 transmittierten Röntgenstrahls 8t ortsaufgelöst und entsprechend der vorbereiteten Energie-Intervalle einschließlich des Referenz-Intervalls ΔE_{R1} energieaufgelöst durchgeführt. Aus der Referenz-Intensität I₀ und der von Testobjekt 16 transmittierten Intensität I werden nun in einem Schritt S5 Werte einer Absorptionsfunktion a für jedes zu kalibrierende Energie-Intervall ΔEⱼ berechnet. Zudem werden im nächsten Schritt S6 für das Referenz-Intervall ΔE_{R1} die Werte der Absorptionsfunktion A berechnet. Daraufhin wird in einem Schritt S7 für jedes Energie-Intervall ΔEⱼ und jedes Pixel des Röntgendetektors 12 ein Scatter-Plot angefertigt, wobei die Werte a der gemessenen Absorptionsfunktion im zu kalibrierenden Energie-Intervall ΔEⱼ auf der Abszissenachse angetragen werden, und die Werte A des Referenz-Intervalls ΔE_{R1} auf der Ordinatenachse. Anschließend wird in einem Schritt S8 aus den Werten der Punkteschar eine Funktion A(a) ermittelt, welche für das betreffende Pixel die Korrekturfunktion zur Korrektur der Strahlaufhärtung im entsprechenden Energie-Intervall ΔEⱼ ergibt, und hierdurch der Röntgendetektor kalibriert.

Obwohl die Erfindung im Detail durch das bevorzugte Ausführungsbeispiel näher illustriert und beschrieben wurde, ist die Erfindung nicht durch dieses Ausführungsbeispiel eingeschränkt. Andere Variationen können vom Fachmann hieraus abgeleitet werden, ohne den Schutzumfang der Erfindung zu verlassen.

## Patentansprüche

1. Verfahren zur Kalibrierung einer Röntgenmesseinrichtung (10), welche einen spektralsensitiven Röntgendetektor umfasst, wobei
- die Röntgenmesseinrichtung (10) für eine Messung zu einer Auflösung einer Mehrzahl an unterschiedlichen Energie-Intervallen (ΔEⱼ) derart präpariert wird, dass der Energiebereich in eine Mehrzahl von Energie-Intervallen (ΔEⱼ) aufgeteilt wird,
- ein Testobjekt (16) im Strahlengang (14) des Röntgenstrahls (8) positioniert wird,
- das Testobjekt (16) durch den Röntgenstrahl (8t) bestrahlt wird, und dabei mittels der Röntgenmesseinrichtung (10) eine nach den Energie-Intervallen (ΔEⱼ) entsprechend aufgelöste Intensitätsmessung des Testobjekts (16) durchgeführt wird,
- anhand der Intensitätsmessung des Testobjekts (16) eine Absorptionsfunktion (a) des Testobjektes (16) ermittelt wird, **dadurch gekennzeichnet, dass**
- eines der Energie-Intervalle (ΔEⱼ) als ein Referenz-Intervall (ΔE_{R1}, ΔE_{R2}) genutzt wird, bei dem die Absorptionsfunktion (a) über das betreffende Energie-Intervall (ΔEⱼ) hinweg eine vernachlässigbare Energieabhängigkeit aufweist,
- anhand wenigstens eines Wertes der Absorptionsfunktion (a) im Referenz-Intervall (ΔE_{R1}, ΔE_{R2}) eine Korrekturfunktion der Absorptionsfunktion (a) für wenigstens ein weiteres Energie-Intervall ermittelt wird, und
- die Röntgenmesseinrichtung (10) anhand der Korrekturfunktion kalibriert wird.

2. Verfahren nach Anspruch 1,
wobei als Referenz-Intervall (ΔE_{R2}) ein breitbandiges Energie-Intervall (ΔEⱼ) genutzt wird, dessen Untergrenze wenigstens den Wert von 60% der Maximalenergie (Eₘₐₓ) des Spektrums des Röntgenstrahls (8) beträgt.

3. Verfahren nach Anspruch 2,
wobei als Referenz-Intervall (ΔE_{R2}) ein für den energieaufgelösten Normalbetrieb der Röntgenmesseinrichtung (10) vorgesehenes Energie-Intervall (ΔEⱼ) gewählt wird.

4. Verfahren nach einem der vorhergehenden Ansprüche,
wobei als Referenz-Intervall (ΔE_{R1}) ein schmalbandiges Energie-Intervall (ΔEⱼ) genutzt wird, dessen spektrale Breite höchstens 1/8 der Maximalenergie (Eₘₐₓ) des Spektrums des Röntgenstrahls (8) beträgt.

5. Verfahren nach Anspruch 4,
wobei für den energieaufgelösten Normalbetrieb der Röntgenmesseinrichtung (10) die Nutzung des Referenz-Intervalls (ΔE_{R1}) aufgehoben wird.

6. Verfahren nach einem der vorhergehenden Ansprüche,
wobei ortsaufgelöst Werte der Absorptionsfunktion (a) für das Referenz-Intervall (ΔE_{R1}, ΔE_{R2}) gegen Werte der Absorptionsfunktion (a) für das wenigstens eine weitere Energie-Intervall (ΔEⱼ) aufgetragen werden, und wobei hieraus die Absorptionsfunktion (a) für das Referenz-Intervall (ΔE_{R1}, ΔE_{R2}) in Abhängigkeit der Absorptionsfunktion (a) für das wenigstens eine weitere Energie-Intervall (ΔEⱼ) als Korrekturfunktion im betreffenden Energie-Intervall (ΔEⱼ) ermittelt wird.

7. Verfahren nach einem der vorhergehenden Ansprüche,
wobei für die Intensitätsmessung des Testobjekts (16) die Röntgenmesseinrichtung (10) bezüglich des Testobjekts (16) eine Rotationsbewegung durchführt.

8. Verfahren nach einem der vorhergehenden Ansprüche,
wobei als Röntgenmesseinrichtung (10) ein quantenzählender Röntgendetektor (12) kalibriert wird.

9. Verfahren nach einem der vorhergehenden Ansprüche,
wobei das Referenz-Intervall (ΔE_{R1}, ΔE_{R2}) über wenigstens eine Spannungsstufe in einem Signalverstärker der Röntgenmesseinrichtung (10) präpariert wird.

10. Bildgebendes medizinisches Gerät (1) mit wenigstens einer Röntgenquelle (6) zur Erzeugung eines Röntgenstrahls (8) und einer einen sprektralsensitiven Röntgendetektor umfassenden Röntgenmesseinrichtung (10), welches zur Kalibrierung der Röntgenmesseinrichtung (10) gemäß dem Verfahren nach einem der vorhergehenden Ansprüche eingerichtet ist.

11. Bildgebendes medizinisches Gerät nach Anspruch 10, welches als ein Computertomograph ausgebildet ist.

12. Bildgebendes medizinisches Gerät nach Anspruch 10, welches als ein C-Bogen-Gerät ausgebildet ist.

## Claims

1. Method for calibrating an X-ray measuring facility (10) which comprises a spectrally sensitive X-ray detector, wherein
- the X-ray measuring facility (10) is prepared for measurement for a resolution of a plurality of different energy intervals (ΔEⱼ) such that the energy range is divided into a plurality of energy intervals (AEⱼ),
- a test object (16) is positioned in the beam path (14) of the X-ray beam (8),
- the test object (16) is irradiated by the X-ray beam (8t), and in the process an intensity measurement of the test object (16) appropriately resolved according to the energy intervals (ΔEⱼ) is carried out by means of the X-ray measuring facility (10),
- an absorption function (a) of the test object (16) is determined using the intensity measurement of the test object (16), **characterised in that**
- one of the energy intervals (ΔEⱼ) is used as a reference interval (ΔE_{R1}, ΔE_{R2}) in which the absorption function (a) has a negligible energy dependency over the relevant energy interval (AEⱼ),
- a correction function of the absorption function (a) is determined for at least one further energy interval using at least one value of the absorption function (a) in the reference interval (ΔE_{R1}, ΔE_{R2}), and
- the X-ray measuring facility (10) is calibrated using the correction function.

2. Method according to claim 1,
wherein a broadband energy interval (ΔEⱼ) is used as a reference interval (ΔE_{R2}), whose lower limit has at least the value of 60% of the maximum energy (Eₘₐₓ) of the spectrum of the X-ray beam (8).

3. Method according to claim 2,
wherein an energy interval (ΔEⱼ) provided for energy-resolved normal operation of the X-ray measuring facility (10) is selected as the reference interval (ΔE_{R2}).

4. Method according to one of the preceding claims,
wherein a narrowband energy interval (ΔEⱼ) is used as the reference interval (ΔE_{R1}), whose spectral width is at most 1/8 of the maximum energy (Eₘₐₓ) of the spectrum of the X-ray beam (8).

5. Method according to claim 4,
wherein the use of the reference interval (ΔE_{R1}) is suspended for energy-resolved normal operation of the X-ray measuring facility (10).

6. Method according to one of the preceding claims,
wherein spatially resolved values of the absorption function (a) for the reference interval (ΔE_{R1}, ΔE_{R2}) are plotted against values of the absorption function (a) for the at least one further energy interval (AEⱼ), and
wherein, from this, the absorption function (a) for the reference interval (ΔE_{R1}, ΔE_{R2}) is determined in the relevant energy interval (AEⱼ), depending on the absorption function (a) for the at least one further energy interval (AEⱼ), as a correction function.

7. Method according to one of the preceding claims,
wherein the X-ray measuring facility (10) carries out a rotational movement in respect of the test object (16) for the intensity measurement of the test object (16).

8. Method according to one of the preceding claims,
wherein a quantum-counting X-ray detector (12) is calibrated as the X-ray measuring facility (10).

9. Method according to one of the preceding claims,
wherein the reference interval (ΔE_{R1}, ΔE_{R2}) is prepared over at least one voltage level in a signal amplifier of the X-ray measuring facility (10).

10. Imaging medical device (1) having at least one X-ray source (6) for generating an X-ray beam (8) and an X-ray measuring facility (10) comprising a spectrally sensitive X-ray detector, said X-ray measuring facility (10) being designed for calibrating the X-ray measuring facility (10) according to the method as claimed in one of the preceding claims.

11. Imaging medical device according to claim 10, which is designed as a computed tomography apparatus.

12. Imaging medical device according to claim 10, which is designed as a C-arm device.

## Revendications

1. Procédé d'étalonnage d'un dispositif (10) de mesure de rayons X, qui comprend un détecteur de rayons X sensible à un spectre, dans lequel
- on prépare le dispositif (10) de mesure de rayons X pour une mesure à une résolution d'une pluralité d'intervalles (ΔEⱼ) d'énergie différents, de manière à ce que la plage d'énergie soit répartie en une pluralité d'intervalles (ΔEⱼ) d'énergie,
- on met l'objet (16) de test en position dans le trajet (14) du faisceau (8) de rayons X,
- on soumet l'objet (16) de test au faisceau (8t) de rayons X et on effectue ainsi, au moyen du dispositif (10) de mesure de rayons X, une mesure d'intensité, résolue conformément aux intervalles (ΔEⱼ) d'énergie,
- à l'aide de la mesure d'intensité de l'objet (16) de test, on détermine une fonction (a) d'absorption de l'objet (16) de test, **caractérisé en ce que**
- on utilise l'un de intervalles (ΔEⱼ) d'énergie comme intervalle (ΔE_{R1}, ΔE_{R2}) de référence pour lequel la fonction (a) d'absorption a, sur l'intervalle (ΔEⱼ) d'énergie concerné une dépendance à l'énergie négligeable,
- à l'aide d'au moins une valeur de la fonction (a) d'absorption, dans l'intervalle (ΔE_{R1}, ΔE_{R2}) de référence, on détermine une fonction de correction de la fonction (a) d'absorption pour au moins un autre intervalle d'énergie, et
- on étalonne le dispositif (10) de mesure de rayons X à l'aide de la fonction de correction.

2. Procédé suivant la revendication 1,
dans lequel on utilise comme intervalle (ΔE_{R2}) de référence un intervalle (ΔEⱼ) d'énergie à bande large, dont la limite inférieure est égale au moins à la valeur de 60% de l'énergie (Eₘₐₓ) maximum du spectre du faisceau (8) de rayons X.

3. Procédé suivant la revendication 2,
dans lequel on choisit comme intervalle (ΔE_{R2}) de référence un intervalle (ΔEⱼ) d'énergie prévu pour le fonctionnement normal résolu en énergie du dispositif (10) de mesure de rayons X.

4. Procédé suivant l'une des revendications précédentes,
dans lequel on utilise comme intervalle (ΔE_{R1}) de référence un intervalle (ΔEⱼ) d'énergie à bande étroite, dont la largeur spectrale est égale au plus au 1/8^{ème} de l'énergie (Eₘₐₓ) maximum du spectre du faisceau (8) de rayons X.

5. Procédé suivant la revendication 4,
dans lequel, pour le fonctionnement normal résolu en énergie du dispositif (10) de mesure de rayons X, on supprime l'utilisation de l'intervalle (ΔE_{R1}) de référence.

6. Procédé suivant l'une des revendications précédentes,
dans lequel on porte des valeurs à résolution dans l'espace de la fonction (a) d'absorption pour l'intervalle (ΔE_{R1}, ΔE_{R2}) de référence par rapport à des valeurs de la fonction (a) d'absorption pour le au moins un autre intervalle (ΔEⱼ) d'énergie, et
dans lequel on en détermine la fonction (a) d'absorption pour l'intervalle (ΔE_{R1}, ΔE_{R2}) de référence en fonction de la fonction (a) d'absorption pour le au moins un autre intervalle (ΔEⱼ) d'énergie comme fonction de correction dans l'intervalle (ΔEⱼ) d'énergie concerné.

7. Procédé suivant l'une des revendications précédentes,
dans lequel, pour la mesure d'intensité de l'objet (16) de test, le dispositif (10) de mesure de rayons X effectue un mouvement de rotation par rapport à l'objet (16) de test.

8. Procédé suivant l'une des revendications précédentes,
dans lequel on étalonne, comme dispositif (10) de mesure de rayons X, un détecteur (12) de rayons X comptant les quantas.

9. Procédé suivant l'une des revendications précédentes,
dans lequel on prépare l'intervalle (ΔE_{R1}, ΔE_{R2}) de référence sur au moins un étage de tension dans un amplificateur de signal du dispositif (10) de mesure de rayons X.

10. Appareil (1) médical d'imagerie, comprenant au moins une source (6) de rayons X pour produire un faisceau (8) de rayons X et un dispositif (10) de mesure de rayons X comprenant un détecteur de rayons X sensible à un spectre, qui est conçu pour l'étalonnage du dispositif (10) de mesure de rayons X par le procédé suivant l'une des revendications précédentes.

11. Appareil médical d'imagerie suivant la revendication 10, qui est constitué sous la forme d'un tomographe à ordinateur.

12. Appareil médical d'imagerie suivant la revendication 10, qui est constitué sous la forme d'un appareil à arceau en C.
